# EUROPEAN PATENT APPLICATION

(11) **EP 3 031 791 A1**
(43) Date of publication of application: **15.06.2016**
(21) Application number: 14197236.4
(22) Date of filing: 10.12.2014
(51) Int. Cl.: C07C 17/02, C07C 51/58

(54) **Halogenation process of 1,1-dihaloethene**

(71) Applicant: Solvay SA, 1120 Bruxelles (BE)
(72) Inventor: The designation of the inventor has not yet been filed
(74) Representative: Mross, Stefan P.M.

(57) **Abstract**

The present invention concerns a halogenation process, in which a 1,1-dihaloethene CX2=CH2 (I) with at least one halogenation agent. The invention concerns further a process for the manufacture of chlorodifluoroacetic acid chloride (CDFAC), which comprise the steps of (a) chlorination of VF2, and (b) an oxidation process. Another object of the present invention is a process for the manufacture of agriculturally or pharmaceutically active compounds, comprising the halogenation and/or oxidation process.

## Description

The present invention concerns a halogenation process, in which a 1,1-dihaloethene CX2=CH2 (I) with at least one halogenation agent. The invention concerns further a process for the manufacture of chlorodifluoroacetic acid chloride (CDFAC), which comprise the steps of (a) chlorination of VF2, and (b) an oxidation process. Another object of the present invention is a process for the manufacture of agriculturally or pharmaceutically active compounds, comprising the halogenation and/or oxidation process.

In a preferred embodiment, in the process according to the invention, X is Cl, and the 1,1-dihaloethene (I) is 1,1-difluoroethene (VF2).

In the halogenation process according to the present invention, (I) is reacted with at least one halogenation agent. In a preferred aspect of the invention, the at least one halogenation agent is elemental halogen, preferably elemental chlorine. Other halogenation agents can also be used.

In a general aspect, the process comprises at least one halogenation reaction which is selected from the group consisting of free radical halogenation and halogenation via ionic intermediates.

In one embodiment, the process comprises one halogenation reaction. Often, in this halogenation reaction, which preferably is a chlorination reaction, elemental halogen, preferably X'2, wherein X' is selected from F, Cl and Br, and preferably is Cl, is added to the double bond of (I), wherein preferably (I) is VF2.

In one aspect, the process according to the present invention can be performed batch-wise. In a more preferred aspect, the process is performed continuously.

In one embodiment, which is preferred, the process comprises more than one, preferably two, halogenation reactions which are selected from the group consisting of free radical halogenation and halogenation via ionic intermediates. In a preferred aspect, the process comprises a first halogenation reaction, which is the addition of two halogen atoms X', wherein X' is selected from F, Cl, Br and I, preferably chlorine, to the double bond of (I), preferably VF2, by reaction with at least one halogenation agent, preferably elemental chlorine. This step yields CX2X'-CH2X' (II). When is X is F and X' is Cl, the product is CF2C1-CClH2, which is often denoted as "132b". This first halogenation reaction often is an ionic reaction which proceeds via a halonium intermediate. The process comprises in this embodiment a second halogenation reaction, in which the intermediary CX2X'-CH2X' (II), preferably CF2Cl-CClH2 (132b), is halogenated further by replacement of one hydrogen atom by a halogen atom X", wherein X" is selected from F, Cl, Br and I, preferably chlorine, to form CX2X'-CHX'X" (III). When F is X, X' is Cl and X" is chlorine, the second product is CF2Cl-CHCl2, often denoted as "122". Generally, this second halogenation reaction is a free radical halogenation reaction. The process comprising two chlorination reaction, in which 122 is formed from VF2 through the intermediate 132b, is preferred. By suitable selection of reaction times and molar ratio of Cl2:VF2, the formation of by-products, such as 122 (1,2,2-trichloro-1,1-difluoroethane), can also be controlled. In one aspect, the process according to this embodiment can be performed batch-wise. In a more preferred aspect, the process according to this embodiment is performed continuously.

In one preferred aspect X' and X" are of the same halogen species, more preferably both are Cl.

In one embodiment of the present invention, the at least one halogenation agent is provided to the process in a molar ratio such that from 2 to 4 atoms of halogen are provided to the process per mole (I), preferably (I) is VF2. Often, the at least one halogenation agent is elemental halogen X'2, wherein X' is selected from the group consisting of F, Cl and Br, preferably Cl. In one aspect of the present invention, the molar ratio of X'2:(I) often equal to or higher than 1:1. Often, the molar ration of X'2:(I) is equal to or higher than 1.05, more preferably equal to or higher than 1.1, and most preferably equal to or higher than 1.15. Generally, the molar ratio of X'2:(I) often equal to or lower than 1.5:1. Often, the molar ration of X'2:(I) is equal to or lower than 1.45:1, more preferably equal to or lower than 1.4:1, and most preferably equal to or lower than 1.3:1. In a preferred aspect, only one halogenation agent, which is preferably elemental chlorine, is added to the process according to the present invention.

In one embodiment of the present invention, the process is carried out under irradiation with UV light. In a preferred aspect, the UV light has a wavelength of λ≥280 nm. For irradiation, it is advantageous to use radiation lamps (e.g., Philips fluorescent tubes) that only emit (UV) light of a wavelength at or above 280 nm (λ≥280 nm). In such case, it is possible to irradiate through quartz glass. In a preferred aspect, the at least one halogenation agent is elemental halogen, preferably chlorine, and the lamps emit in the absorption range of the elemental halogen, preferably chlorine. Alternatively, it is possible to use radiation lamps (e.g., Hg medium or high-pressure discharge lamps), which also emit some lines in the range below 280 nm (λ<280 nm), i.e., that filters out the shorter wave radiation component of λ<280 nm. Well suited are, for instance, borosilicate glasses. This type of glass typically contains 7 to 13 percent B2O3, 70 to 80 percent SiO2, furthermore 2 to 7 percent Al2O3 and 4 to 8 percent Na2O+K2O and 0 to 5 percent alkaline-earth metal oxides. Known trademarks for borosilicate glasses are Duran™, Pyrex™ and Solidex™. It is of course also possible to proceed by using on the one hand a radiation lamp that emits light above the indicated wavelengths and, in addition, glasses that are transparent for light above the indicated wavelength (i.e., that are non-transparent for light below the indicated wavelength).

In one embodiment of the present invention, the reaction time is from 3 to 90 hours. The reaction time is often dependent on the molar ratio of the at least one halogenation agent to (I) and the desired reaction product. In a preferred aspect, wherein one halogenation agent is provided to the reaction, which is elemental chlorine, and (I) is VF2, and the molar ratio of Cl2:VF2 is from 1:1 to equal to or lower than 1.2:1, and the preferred reaction product is 122, the reaction time preferably is from 400 to 1300 minutes. In another preferred aspect, wherein one halogenation agent is provided to the reaction, which is elemental chlorine, and (I) is VF2, and the molar ratio of Cl2:VF2 is from 1.21:1 to equal to or lower than 1.4:1, and the preferred reaction product is 122, the reaction time preferably is from 2300 to 3500 minutes. In yet another aspect, wherein one halogenation agent is provided to the reaction, which is elemental chlorine, and (I) is VF2, and the molar ratio of Cl2:VF2 is from 1.41:1 to equal to or lower than 1.6:1, and the preferred reaction product is 122, the reaction time preferably is from 2800 to 3500 minutes.

The reaction can be performed in the gas phase or in the liquid phase, wherein the liquid phase in preferred. The solvent preferably is carried out in at least one inert solvent, preferably selected from the group consisting of CC14 and HCFC-132b. The halogenation process generally is performed at a temperature of from 10 to 40°C, preferably of from 15 to 30°C, more preferably of from 18 to 25°C.

The invention also concerns a process for the manufacture of chlorodifluoroacetic acid chloride (CDFAC), which comprise the steps of (a) a halogenation process according to present invention, wherein the at least one halogenation agent is a chlorination agent, preferably elemental chlorine, and wherein the process preferably comprises two consecutive steps to yield a fraction comprising 122, and (b) an oxidation process. In a preferred aspect, the oxidation process according to step (b) is a photooxidation process. In one aspect, the fraction comprising 122 obtained by (a) is submitted to at least one intermediary purification step before (b), wherein the one or more intermediary steps provide a fraction enriched in 122. The at least one intermediary purification step often is at least one distillation step, wherein the fraction enriched in 122 can be obtained as bottom or top product of a column distillation. In a further aspect, the step a) and b) can be performed as one-pot process in immediate consecutive order. The manufacture of CDFAC can be performed batch-wise or continously, which applies to each individual step a) and b), or wherein a) and b) are performed in a one-pot process setup.

In one embodiment, the process for the manufacture of chlorodifluoroacetic acid chloride (CDFAC) comprises at least one purification step after step (b) to obtain at least a fraction rich in CDFAC.

The invention concerns further a process for the manufacture of agriculturally or pharmaceutically active compounds, comprising the halogenation process, in particular the chlorination process, as outlined above. In one embodiment of the invention, VF2 is chlorinated to yield a fraction comprising CClF2-CClH2 (132b) and F2ClC-CHCl2 (122). The fraction can be used directly or, preferably, after at least one purification step to yield a fraction enriched in 122, in an oxidation process, in particular a photooxidation process, to yield a fraction comprising CDFAC. The fraction comprising CDFAC can suitably be used in the manufacture of agriculturally or pharmaceutically active compounds, as described, for example, in WO2010037688 and WO2012/25469 wherein CDFAC is converted to fluorosubstituted-3-oxo-alcanoic acids, which can further be converted into intermediates for agriculturally active compounds.

Should the disclosure of publications which are incorporated herein by reference conflict with the description of the present application to the extent that it may render a term unclear, the present description shall take precedence.

The examples which follow are intended to illustrate the present invention without, however, limiting the scope thereof.

### Experiments

VF2 and Cl2 were reacted at a temperature of 20°C in the given solvent and in the given ratio under irradation with UV light with a wavelength of λ≥280 nm in a 0.9 Liter-reactor. The resulting fraction was analyzed with GC.

| Exp No. | Molar ratio Cl2:VF2 | Time (min) | 132b in product (weight %) | 122 in product (weight %) | 122a in product (weight %) | Ratio 132b:122 in product (mol/mol) | solvent |
|---|---|---|---|---|---|---|---|
| 1 | 1:1 | 428 | 57,9 | 37,8 | 4,3 | 1,2 | CCl4 |
| 2 | 1:1 | 1020 | 64,1 | 32,1 | 3,8 | 2,5 | CCl4 |
| 3 | 1:1 | 1815 | 42,3 | 13,1 | 0,97 | 4,1 | CCl4 |
| 4 | 1.3:1 | 1908 | 47,8 | 13,5 | 0,9 | 4,5 | CCl4 |
| 5 | 1.3:1 | 2433 | 49,6 | 27,6 | 2,2 | 2,3 | CCl4 |
| 6 | 1.3:1 | 2970 | 57,5 | 38,8 | 3,7 | 1,9 | CCl4 |
| 7 | 1.5:1 | 3178 | 50,45 | 36,2 | 3,9 | 1,7 | CCl4 |
| 8 | 1.5:1 | 3730 | 44,7 | 42,6 | 6.3 | 1,3 | CCl4 |
| 9 | 1.5:1 | 3906 | 43,6 | 45 | 6,9 | 1,2 | CCl4 |

## Claims

1. Process for the halogenation of 1,1-dihaloethene CX2=CH2 (I), wherein 1,1-dihaloethene is reacted with at least one halogenation agent.

2. Process according to claim 1, wherein the X is X, and the 1,1-dihaloethene is 1,1-difluoroethene (VF2).

3. Process according to claim 1 or 2, wherein the at least one halogenation agent is a chlorination agent.

4. Process according to claim 3, wherein the at least one chlorination agent is elemental chlorine.

5. Process according to anyone of claims 1 to 4, wherein the process comprises at least one halogenation reaction which is selected from the group consisting of free radical halogenation and halogenation via ionic intermediates.

6. Process according to anyone of claims 1 to 5, wherein the at least one halogenation agent is provided to the process in a molar ratio such that from 2 to 4 atoms of halogen are provided to the process per mole (I).

7. Process according to anyone of claims 1 to 6, wherein the process is carried out under irradiation with UV light.

8. Process according to claim 7, wherein the UV light has a wavelength of λ≥280 nm.

9. Process according to anyone of claims 1 to 8, wherein the reaction time is from 3 to 90 hours.

10. Process for the manufacture of chlorodifluoroacetic acid chloride (CDFAC), which comprise the steps of (a) the process according to claims 2 and 3 or 2 and 4 in combination with anyone of claims 5 to 8, and (b) an oxidation process.

11. Process according to claim 10, wherein the oxidation process is a photooxidation process.

12. Process according to claim 10 or 11, wherein the process comprises further an intermediary purification step after step (a) such that F2ClC-CHCl2 (122) is enriched in the crude product fraction obtained by step (a).

13. Process according to anyone of claims 10 to 12, which further comprises at least one purification step after step (b) to obtain at least a fraction rich in CDFAC.

14. A process for the manufacture of agriculturally or pharmaceutically active compounds, comprising the process according to any one of claims 1 to 13.
